# EUROPÄISCHE PATENTSCHRIFT

(11) **EP 1 317 886 B1**
(45) Veröffentlichungstag und Bekanntmachung des Hinweises auf die Patenterteilung: **20.06.2007**
(21) Anmeldenummer: 02090411.6
(22) Anmeldetag: 26.04.1999
(51) Int. Cl.: A24D 3/16, B01D 39/00, B01D 71/02

(54) **Verwendung von mikronisierten Zeolithen als Filterstoffe**
Use of micronised zeolites as filter material
Utilisation de zéolites micronisées comme matériel filtrant

(43) Veröffentlichungstag der Anmeldung: 11.06.2003
(62) Teilanmeldung aus: 99913546.0
(73) Patentinhaber: Lelas, Tihomir, 10040 Zagreb (HR)
(72) Erfinder: Lelas, Tihomir, 10040 Zagreb (HR)
(74) Vertreter: Omsels, Hermann-Josef

(56) Entgegenhaltungen:
- EP-A- 0 740 907
- US-A- 3 327 718
- US-A- 4 038 992
- US-A- 5 612 522
- US-A- 5 723 397
- US-A- 5 871 650

## Beschreibung

Die Erfindung betrifft die Verwendung mikronisierter Zeolithe als Filterstoff.

Eine Vorrichtung, welche Zeolithe derart zerkleinern kann, dass deren Wirksamkeit verbessert wird, ist bereits aus der DE 197 55 921.2 bekannt. Die dortige Erfindung beschreibt ein Verfahren zur Verbesserung der Wirksamkeit von Wirkstoffen, die mindestens aus Mineralstoffen bestehen, indem diese Wirkstoffe einer tribomechanischen Aktivierung unterzogen werden, bei der die Oberfläche der behandelten Wirkstoffe vergrößert und deren Struktur zur Vergrößerung des chemischen Potentials destabilisiert wird. Die Aktivierung der Mineralstoffe geschieht dadurch, dass in die Integrität der Kristallgitter eingegriffen wird, wodurch sich eine Art Beschädigung ergibt, die sich wiederum in Form einer Aktivierung, beispielsweise auch elektrischer Art, bemerkbar macht. Die DE 197 55 921.2 sieht dabei als vorteilhaft die Behandlung von Zeolithen an, die dort zum heilsamen Verzehr für Menschen beschrieben werden; auch Calcite für den Agrarbereich werden erwähntdassDie durch die aus der DE 197 55 921.2 bekannte Vorrichtung zu erzielende Mikronisierung liegt bei 20 µm pro Teilchen, wobei nur ca. 78 % aller Teilchen diese Größenordnung erreichten.

Aus der US 5,723,397 A und der US 5,871,650 wird die Verwendung von Zeolithen in Membran oder Filtern offenbart. Dabei werden auch Zeolithe mit einem Korngrößendurchmesser von weniger als 0,5 µm verwendet. Eine Aktivierung der Zeolithe durch einen speziellen Zerkleinerungsprozess wird in beiden Dokumenten jedoch nicht offenbart.

Die EP 0 740 907 A beschreibt wie die US 3,327,718 A und die US 4,038,992 A die Verwendung von Zeolithmineral in Zigarettenfiltern.

Ausgehend von diesem Stand der Technik ist es daher Aufgabe der vorliegenden Erfindung, hochaktive Zeolithe zur Verwendung als Filterstoff zur Verfügung zu stellen, insbesondere für Zigarettenfilter.

Gelöst wird diese Aufgabe durch die im Patentanspruch 1 und 2 aufgeführten Merkmale, nämlich dadurch, dass mikroniserte Zeolithe mit einem Korngrößendurchmesser von weniger als 0,5 µm als Filterstoff verwendet werden, welche durch Rotoren, die aus Scheiben bestehen, auf denen die Ventilatorschaufeln einseitig befestigt angeordnet sind und wobei die Ventilatorschaufeln mit den Kränzen verbunden sind und in korrespondierende Kanäle auf der jeweils gegenüberliegenden Rotorenscheibe durchgreifen, die einen Materialdurchgang unter den Ventitatorschaufeh verhindern. Durch diese erfindungsgemäßen Maßnahmen wird eine wesentlich effizientere Mikronisierung erreicht unter gleichzeitiger Schonung der Vorrichtung selbst als sie im Stand der Technik bisher bekannt war. Erreicht wird dies dadurch, dass durch die Kanäle die Abnutzung der Ventilatorschaufeln selbst minimiert wird, indem bewusst ein Verbleiben von mikronisiertem Material innerhalb der Kanäle in Kauf genommen wird, was wiederum zu einem erhöhten Widerstand an den Ventilatorschaufeln selbst führt und damit letztlich zu einem erhöhten Grad der Mikronisierung.

Die Vorteile der Zerkleinerung lassen sich wie folgt darstellen:
■ Diese Feinmahlung wird durch kontrollierte Luftströmungen erzielt, die durch die Anwendung von neuartig konstruierten Ventilatorschaufeln hervorgerufen werden.
■ Die Kanäle, in welchen die Ventilatorschaufeln durchgreifen, bilden ein geschlossenes Labyrinthsystem für die Materialverarbeitung, welches die Bewegung des Materials, das der Verarbeitung unterzogen ist, so kontrolliert, dass die Körnchen nicht ohne Schlag- und Reibungswirkung neben den Ventilatorschaufeln unverarbeitet vorbeifliegen, womit die Effizienz der Verarbeitung optimiert wird.
■ Die zu erzielende Mikronisierung liegt bei 98,72 % aller Teilchen unter 4,3 µ. Ein Anteil von 28,36 % aller Teilchen weist sogar einen Durchmesser von unter 0,5 µm auf. Mit keinem herkömmlichen Verfahren oder einer bekannten Vorrichtung konnten derartige Ergebnisse erzielt werden.

Das durch die erfindungsgemäße Vorrichtung mikronisierte Material weist vielerlei Vorteile für unterschiedlichste Verwendungsmöglichkeiten auf:

Die neuartige Vorrichtung ruft bei mineralischen Rohstoffkomponenten diverse chemische und chemisch-physikalische Veränderungen hervor. Die Effekte, welche durch dynamische Reibungsprozesse entstehen, verleihen diesen Mineralen neue Eigenschaften, die sich bei der Herstellung diverser Produkte technologisch und kommerziell nutzen lassen.

Aus der Gruppe der Zeolithe hat sich das Mineral Heulandit/Klinoptylolith aufgrund seiner Eigenschaften als vorteilhaft herausgestellt, nämlich aufgrund seiner Fähigkeit zur Wasseraufnahme, seiner Selektivität und lonenaustauschkapazität, sowie seiner chemischen Zusammensetzung, welche zeigte, daß dieses Mineral für den menschlichen Genuß absolut unbedenklich ist. Die mineralogischen und chemischen Eigenschaften von Klinoptilolithen wurden untersucht und stellen sich wie folgt dar:

**Tabelle 1**

| **Komponente** | **Anteil [%]** | |
|---|---|---|
| | **von** | **bis** |
| SiO₂ | 61,96 | 67,17 |
| TiO₂ | 0,15 | 0,32 |
| Al₂O₃ | 12,46 | 15,12 |
| Fe₂O | 0,98 | 2,05 |
| MnO | spur | 0,05 |
| MgO | 1,30 | 1,96 |
| CaO | 3,03 | 4,35 |
| Na₂O | 0.70 | 1,11 |
| K₂O | 0,78 | 1,32 |
| H₂O bei 100°C | 4,05 | 4,74 |
| H₂O bei 1000°C | 7,56 | 9,56 |

Der Kalziumgehalt dieses Minerals deutet darauf hin, daß es sich dabei um ein Kalziumzeolith handelt, welches in einer tuffartigen Struktur gebildet ist, d. h. es handelt sich hier um eine Art des Minerals Klinoptilolith mit den Eigenschaften der Heulanditgruppe. Das gemessene Raumgewicht des untersuchten Minerals Klinoptiloithes bewegt sich in dem Bereich von 1,41 bis 1,43 g/cm³. Difraktometrische und thermo-gravimetrische Untersuchungen zeigten, daß in allen untersuchten Mustern ungefähr gleicher Zeolithgehalt besteht. Die Ergebnisse der Röntgenuntersuchungen zeigen die Anwesenheit von folgenden Mineralsorten: Heulandithe (Klinoptilolithe), sowie in weiterer Folge im wesentlichen Quarz, Sand, Plagioklass und in einer geringen Menge auch Biothythe. Die mikroskopische Untersuchung mittels Elektronenmikroskop zeigte, daß die Materialstruktur aus feinen Tuffkömern besteht, welche eine homogene Isotropmasse, praktisch die Zeolithmaterie, darstellt. Der Gehalt dieser Materie bewegt sich grundsätzlich immer im Rahmen zwischen 70 % bis 85 %. Es ist in weiterer Folge die Anwesenheit von eckigen Quarzsegmenten festgestellt worden, sowie plagioklasse Sandkörner, welche in der Regel eine durchschnittliche Korngröße von 60 µm aufweisen. Die Untersuchung des Schmelzpunktes an 10 Mustern hat gezeigt, daß Klinoptilolithe bei einer Temperatur von 1260 - 1280° C schmelzen. Die festgestellte Härte laut Moss beträgt 3 - 3,5. Der Glühverlust beträgt:

**Tabelle 2**

| | | Glühverlust [Gew.-%] |
|---|---|---|
| H₂O | bei 100 °C | 3,34 bis 3,36 |
| H₂O | bei 300 °C | 5,42 bis 5,51 |
| H₂O | bei 500 °C | 2,60 bis 2,64 |
| H₂O | bei 1000 °C | 2,50 bis 2,51 |
| Insgesamt | | 13,86 bis 13,92 |

Ergebnisse der Absorbtionsuntersuchungen:

**Tabelle 3**

| Zeitraum | Wasser [Gew.-%] | | Benzol [Gew.-%] | |
|---|---|---|---|---|
| [h] | von | bis | von | bis |
| 1 | 4,61 | 4,62 | 7,64 | 7,65 |
| 2 | 8.74 | 8,75 | 9,33 | 9,33 |
| 3 | 10,75 | 10,77 | 9,45 | 9,47 |
| 4 | 11,10 | 11,11 | 9,51 | 9,54 |
| 17 | 13,44 | 13,45 | 9,54 | 9,54 |

Weitere Eigenschaften des untersuchten Klinoptilolithes:

| | | |
|---|---|---|
| 1. Druckfestigkeit | a)bei trockenem Zustand | max. 426 kg/cm³ |
| | | min. 361 kg/cm³ |
| | | mittel 391 kg/cm³ |
| | b) mit Wasser gesättigten Zustand | |
| | | max. 360 kg/cm³ |
| | | min. 253 kg/ cm³ |
| | | mittel 292 kg/cm³ |
| 2. Wasseraufnahme | | 23,35 Gew.-% |
| 3. Raumgewicht | | 1,37 g/cm³ |

Die Untersuchungsergebnisse der elektrischen Leitfähigkeit zeigten, daß die erfindungsgemäß behandelten Zeolithe bedeutend mehr Wasserstoffionen zu binden vermögen als die unbehandelten Zeolithe. Dies ist die unmittelbare Folge der Unterschiede in der Kristallstruktur der untersuchten Zeolithe, die durch die Feinmahlenung und Mikronisierung zustandegekommen sind. Die folgende Tabelle zeigt Beispiele für die Messungen der Leitfähigkeit und des pH der Suspension bei nicht behandelten und behandelten Zeolithen:

**Tabelle 4**

| Mischzeit der Suspension ^{a} | Konzentration an Zeolith (mg/ml H₂O) | pH^{b} (unaktiviertes Zeolith) | pH^{b} (erfindungsgemäß behandeltes Zeolith) | Leitfähigkeit^{c} (unaktiviertes Zeolith) µS/cm | Leitfähigkeit^{c} (erfindungsgemäß behandeltes Zeolith) µS/cm |
|---|---|---|---|---|---|
| 2 Std. | 20 | 7,35 | 7,48 | 157 | 147 |
| 2 Std. | 40 | 6,45 | 7,22 | 198 | 180 |
| 2 Std. | 60 | 6,81 | 7,35 | 307 | 280 |
| 20 Std. | 40 | 6,67 | 6,90 | 259 | 218 |
| 20 Std. | 80 | 6,96 | 7,40 | | |
| 48 Std. | 80 | 6,87 | 7,44 | | |

| | | | | | |
|---|---|---|---|---|---|
| ^{a} mit Magnetrührer, 70 - 100 RPM; ^{b} pH Messung unmittelbar nach der Zentrifugation (15 Minuten auf 7000 RPM), ^{c} Leitfähigkeitsmessung im Überstand unmittelbar nach der Zentrifugation (15 Minuten auf 7000 RPM), Leitfähigkeit des redestillierten Wassers beträgt 1.8 µS/cm | | | | | |

Die Ergebnis ist, daß der pH-Wert der unbehandelten zu den behandelten Zeolithe steigt, während die Leitfähigkeit abnimmt. Das erfindungsgemäß behandelte Material zeigt überraschende Wirkungen im Hinblick auf die Minimierung gesundheitsschädlicher Inhaltsstoffe von Filterzigaretten.

Zigarettenrauch ist ein sichtbares, verdunstendes Produkt der Tabakverbrennung, der Additive und des Zigarettenpapiers, der gleich nach der Verbrennungszone entsteht und sich wie folgt zusammensetzt:
■ gasförmige Phase (CO, CO₂, O₂, H₂, N₂N-Oxyd, HCN, HCNS, NH₃, Aldehyde, Alkohole usw.),
■ starr-flüssige Phase, zusammengesetzt aus Wasser und in ihr ganz oder teilweise schmelzende Verbindungen (wie Nikotin und weitere Alkaloide), die während des Rauchens destillieren, verdunsten oder verbrennen.

Während der Rauch durch den Tabak zieht, kommt es zur Abkühlung und Kondensation, ein Teil des Rauches gelangt in die Mundhöhle. Wegen der Verringerung der möglichen negativen, gesundheitsschädlichen Folgen des Rauches wird bekanntermaßen als Mundstück der Zigarette ein Zigarettenfilter eingebaut.

Der klassische Filter ist der Zellulose-Acetat-Filter. Um die Effizienz des Filters zu vergrößern (Fähigkeit des Zurückhaltens, der Absorption starrflüssiger und gasförmiger Phasen des Rauches), werden Additive, wie es Aktivkohle, Zeolithe, Silikagel, Magnesiumsilikat und künstliches Hämoglobin sind, verwendet.

Bekannt sind Mehrfachfilter, Papierfilter, Kombinationen mehrerer Filter und Additiva. Die Effizienz zur Reduzierung schädlicher Komponenten eines einfachen (Zellulose-Acetat-) Filters beträgt 20 - 65 %; durch verschiedene neuartig entwickelte Kombinationen, Additiva und Formen der Filter wird die Effizienz auf ca. 75 % angehoben. Ein großer Marktanteil kommt heute schon den sogenannten Leicht-Zigaretten ("Lights" oder "Ultra") zu, die weniger Trockenrauch-Kondensat, Wasser, Nikotin, CO₂ und andere schädliche Komponenten enthalten oder verursachen. Beim Projektieren von Light-Zigaretten nutzt man die Ventilation am Filter (Verdünnung des Rauches), verlängertes Wickelpapier der Filter (skirt tipping) und eine Kombination verschiedener Zigarettenpapiere und verschiedener Tabake. Die Effizienz und die Selektierung des Filters (erhöhte Fähigkeit der Absorption bestimmter Komponenten), sind ein wichtiger Faktor beim Projektieren der Light-Zigaretten. Einer der wichtigen Faktoren bei der Konstruktion des Filters und auch der Zigarette, ist der Saugwiderstand (pressure drop), der wesentlich auf den Geschmack und die Leichtigkeit des Rauchens Einfluß hat. Viele Additive und Konstruktionslösungen vergrößern den Saugwiderstand und ungewollte Effekte beim Rauchen. Dies ist der einfache Grund, warum noch immer beim größten Teil die einfachen Zellulose-Acetat-Filter verwendet werden.

Das erfindungsgemäß behandelte Material erhöht überraschenderweise die Effizienz und Selektion der Filter, sowie ein Anhalten und eine Verbesserung des gewünschten Geschmackes der Zigarette; dabei wird der Saugwiderstand des Filters kaum verändert. Die behandelten Zeolithe weisen eine größere Absorptionstärke auf, was auf die Veränderungen im Kristallgitter zurückzuführen ist.

Vorteilhaft hat sich ein Filterzusatz bei Zellulose-Acetat Filtern als Pulver mit einer Korngöße von 0,2 bis 0,5 µm erwiesen.

Bei der Herstellung der Filter, kann man die erfindungsgemäß behandelten Zeolithe direkt auf die Zellulose-Acetat-Faser oder das Papier bzw. Crest aufbringen - wie einen Zusatz im Hohlraum eines Multifilters, kombiniert mit dem Dualfilter usw.

Filter, die Gegenstand eines kontrollierten Experiments waren, wurden durch Hinzufügen von erfindungsgemäß behandelten Zeolithen direkt auf die Zellulose-Acetat-Faser produziert, nachdem man Plastifikatoren (Triacetin) vor dem Formen der Filterstäbchen im Zylinder eingesetzt hat. Das Pulver wurde durch ein besonders ausgearbeitetes Dosimeter direkt auf die Faser aufgebracht. Dadurch war es möglich, fast einheitlich bis zu 70 mg des erfindungsgemäß behandelten Zeoliths pro Filterstäbchen hinzuzufügen. Diese Art der Zugabe des Pulvers beeinflußt die Arbeit der herkömmlichen Maschinen und das Formen der Filterstäbchen nicht wesentlich, da die Maschinen zur Herstellung der Füterstäbchen einen dafür vorgesehenen Raum im Arbeitsfluß für diese Applikation haben. Durch diese Art der Zugabe des Pulvers ändert sich die Technologie der Stäbchenproduktion nicht wesentlich. Das technologische Verfahren wird nur um den Preis der Additive erhöht.

Die durchschnittlichen Resultate der gemessenen physischen Filterparameter ohne Zusatz von erfindungsgemäß behandelten Zeolithen und mit Zusatz von erfindungsgemäß behandelten Zeolithen, werden nachfolgend dargestellt.

**Tabelle 5**

| Muster | Saugwiderstand | Filtermasse | Zugabe von erfindungsgemäß behandelten Zeolith | |
|---|---|---|---|---|
| | [mm VS] | [mg] | 12 cm Filter | 2 cm Filter |
| 0 | 390 | 721 | 0 | 0 |
| 1 | 392 | 750 | 3,0 | 0,50 |
| 2 | 395 | 765 | 5,0 | 0,83 |
| 3 | 395 | 766 | 7,6 | 1,27 |
| 4 | 410 | 773 | 10,0 | 1,66 |
| 5 | 440 | 782 | 68,0 | 11,30 |

Die Zugabe der Zeolithe hat sich nicht wesentlich auf den Saugwiderstand und auch nicht auf das Gewicht des 12 cm langen Filterstäbchens ausgewirkt.

Die erhaltenen Resultate sind Durchschnitte der Messungen von 500 Filterstäbchen von jedem Muster. Der Anteil des zugegebenen behandelten Zeoliths wurde aufgrund des Unterschiedes der erhaltenen Menge Asche beim Verbrennen der Filterstäbchen im Mufon-Ofen bei 525° C berechnet. Für die Verarbeitung der Zigarette wurde im wesentlichen herkömmliches Filterpapier ohne Ventilation und ein Tabakgemisch, welches bei der Herstellung von full flavour-Zigaretten des Typs American Blend verwendet wird. Die verarbeiteten Zigaretten wurden nach Gewicht und Saugwiderstand sortiert und auf der Rauchmaschine (Borgwaldt RM 20) nach dem ISO-Standard erprobt. Jedes Muster wurde 5 mal auf der Maschine erprobt. Die Menge an Trockenrauch-Kondensat (Total Particule Matter), Nikotin, CO₂, Wasser und Teer wurden bestimmt. Alle Analysen wurden nach dem geltenden ISO-Standard; die Menge an Nikotin im Trockenrauch-Kondensat durch Extraktion mit 2-Propanol und anschließender HPLC durchgeführt.

Die erhaltenen durchschnittlichen Resultate sind in der nachfolgenden Tabelle dargestellt, aus der ersichtlich ist, dass bei Erhöhung des zugegebenen erfindungsgemäß behandelten Zeoliths im Filter, sich der Anteil an Nikotin, Trockenrauch-Kondensat, Teer, Wasser und CO₂ im Hauptstrom des Zigarettenrauches verringert. Gesetzlich sind die Zigarettenhersteller verpflichtet, den Gehalt an Teer und Nikotin in der Zigarette anzugeben. Dies macht die Bedeutung der Wesensart des CO₂ nicht geringer, welches negative Einwirkungen auf den Respiratorzyklus des Organismus hat. Das erfindungsgemäß behandelte Zeolith beseitigt überraschenderweise offenbar selektiv die schädlichen Komponente im Hauptstrom des Zigarettenrauches. Unabhängig von der Menge des Zeoliths, das auf den Filter appliziert wurde, ändert sich der Widerstand der Zigarette nicht wesentlich, aber auch nicht die Verbrennungsgeschwindigkeit. Die Leichtigkeit und Annehmlichkeit des Rauchens wurde beibehalten. Die Degustationsprüfungen haben gezeigt, dass die Muster mit dem größten Anteil an dem erfindungsgemäß behandelten Zeolith (Muster 5 - 11,3 mg und Muster 4 -1,66 mg) einen verbesserten Rauchgeschmack haben und ein hervorgehobenes Aroma aufweisen, so dass sie allgemein angenehmer zu rauchen sind.

**Tabelle 6**

| | | **Muster** | | | | | |
|---|---|---|---|---|---|---|---|
| **Zusammensetzung** | **Einheit** | **0** | **1** | **2** | **3** | **4** | **5** |
| Erfindungsgemäß behandeltes Zeolith | Filter [mg] | 0 | 0,50 | 0,83 | 1,27 | 1,66 | 11,30 |
| Trockenrauch-Kondensat | Zigarette [mg] | 14,8 | 14,2 | 13,5 | 13,2 | 12,9 | 10,8 |
| Nikotin | Zigarette [mg] | 1,03 | 0,98 | 0,94 | 0,93 | 0,91 | 0,75 |
| CO₂ | Zigarette [mg] | 14,98 | 14,37 | 13,61 | 12,46 | 11,86 | 10,90 |
| Wasser | Zigarette [mg] | 1,18 | 1,05 | 1,09 | 0,95 | 0,73 | 0,68 |
| Teer | Zigarette [mg] | 12,6 | 12,2 | 11,5 | 11,3 | 11,3 | 9,4 |
| Tabakmasse in Zigarette | [mg] | 764 | 759 | 770 | 787 | 752 | 764 |
| Anzahl der Züge | Züge/Zigarette | 7,4 | 7,0 | 7,4 | 7,5 | 7,2 | 7,4 |
| Saugwiderstand der Zigarette | [mm VS] | 102 | 102 | 105 | 109 | 108 | 111 |

In der nachfolgenden Tabelle sind die gemessenen durchschnittlichen Werte der Filter nach dem Rauchen dargestellt. Die Menge an Trockenrauch-Kondensat wurde aus dem Differenz der Menge des Produktionsmaterials vor und nach dem Rauchen berechnet. Die Menge des Nikotins wurde durch Extraktion aus dem Filter in 2-Propanol und Wasser durch die Karl-Fisher Methode bestimmt. Die Resultate zeigen, dass die mit dem erfindungsgemäß behandelten Zeolith applizierten Filter, eine erhöhte Fähigkeit der Absorption von Trockenrauch-Kondensat, Nikotin, und Wasser haben, welches die vorangegangenen Messungen und Resultate unterstreicht.

**Tabelle 7**

| | | **Muster** | | | | | |
|---|---|---|---|---|---|---|---|
| **Zusammensetzung** | **Einheit** | **0** | **1** | **2** | **3** | **4** | **5** |
| Erfindungsgemäß behandeltes Zeolith | mg/Filter | 0,00 | 0,50 | 0,83 | 1,27 | 1,66 | 11,30 |
| Trockenrauch-Kondensat | mg/Filter | 13,50 | 13,30 | 13,80 | 14,30 | 14,80 | 15,40 |
| Nikotin | mg/Filter | 0,53 | 0,58 | 0,60 | 0,60 | 0,67 | 0,69 |
| Wasser | mg/Filter] | 1,01 | 1,02 | 1,03 | 1,12 | 1,05 | 1,12 |

Aus diesen Ergebnissen folgt, dass eine Beimengung des erfindungsgemäß behandelten Minerals von 0,5 mg (vgl. Muster 1) vorteilhaft ist, da der Anteil an Trockenrauch-Kondensat abnimmt. Auch geht aus dieser Tabelle hervor, dass ein verstärktes Zurückhalten von Nikotin im Filter zu beobachten ist.

Durch die Applikation des erfindungsgemäß behandelten Zeoliths auf Zellulose-Acetat-Faser von Zigarettenfiltern führt also zu einer verstärkten Retention schädlicher Komponenten des Hauptstromes des Zigarettenrauches, zufriedenstellenden physischen Eigenschaften, ohne aber negativen Einfluß auf die degustativen und Raucheigenschaften einer Zigarette zu bewirken. Der Zusatz von 0,50 mg des erfindungsgemäß behandelten Zeoliths pro Filter beseitigt 4 % Trockenrauch-Kondensat, CO₂ und Nikotin, sowie 11 % Wasser. Ein Zusatz von 11,30 mg vom erfindungsgemäß behandelten Zeoliths pro Filter beseitigt 27 % Trockenrauch-Kondensat, CO₂ und Nikotin, sowie 42 % Wasser aus dem Hauptstrom des Rauches.

Ferner ist vorteilhaft eine weitere Zugabe von Selen (bis 25 µg/Filter) zu dem erfindungsgemäß behandelten Zeolith, da dadurch die Absorption freier Radikale, die durch Verbrennungen entstehen, erhöht wird.

Eine Vorrichtung, welche zur Zerkleinerung des Zeoliths verwendet wird und zu den vorteilhaften Eigenschaften des zerkleinerten Materials führt, wird nachfolgend beschrieben. Es zeigt:
- **Figur 1**: eine schematische Ansicht der erfindungsgemäßen Vorrichtung;
- **Figur 2**: einen vertikalen Schnitt durch die Vorrichtung;
- **Figur 3**: eine Rotorenscheibe;
- **Figur 3a**: einen vertikalen Schnitt durch einen Ausschnitt der zusammengesetzten Vorrichtung,
- **Figur 3b**: eine Ausschnittvergrößerung entlang eines Schnittes A-A gemäß der Fig. 3a;
- **Figur 4**: die schematische Andeutung der Luftströme entlang der Ventilatorschaufeln;
- **Figur 5**: die schematische Andeutung des zerkleinerten Materials entlang der Ventilatorschaufeln;
- **Figur 6**: eine Ventilatorschaufel mit Stift/Kanal im Detail und in mehreren Ansichten **(6a, 6b, 6c);**
- **Figur 7**: schematische Darstellung einer Segmenten-Ventilatorschaufel;
- **Figur 7a**: Ausschnitt entlang eines Schnittes A-A gemäß Figur 7.

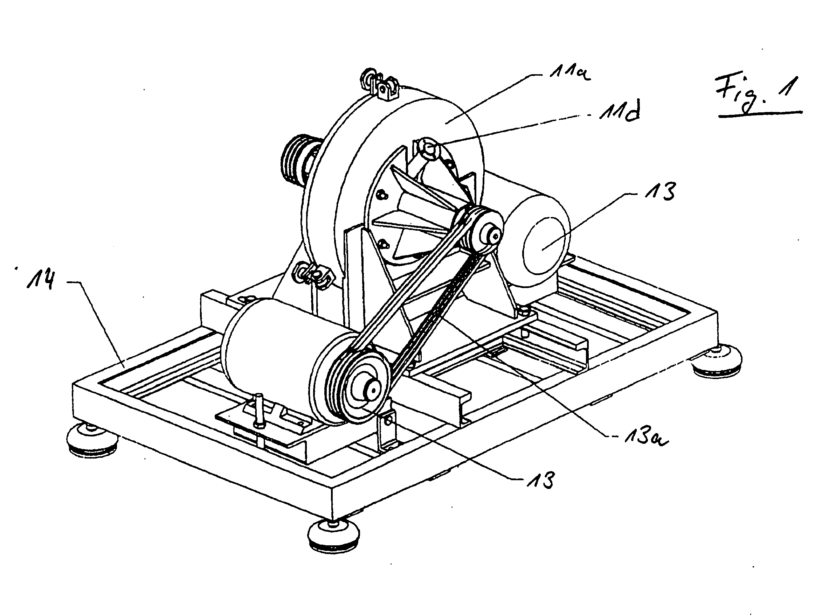

Die Figur 1 zeigt eine Vorrichtung 10 für die Feinmahlung und Mikronisierung, sowie das Homogenisieren von diversen festen und flüssigen Rohstoffkomponenten. Das Prinzip sieht derart aus, dass das Ausgangsmaterial durch die Mitte der Rotoren in den Verarbeitungsraum der Vorrichtung eingesaugt wird. Der Eintritt wird durch die Einwirkung von zentrifugalen Kräften in den Raum zwischen den Ventilatorschaufeln begünstigt und wird aufgrund der dort herrschenden Luftströme beschleunigt; so dass das Material mit dem bereits verarbeiteten Material kollidiert. Das Ausgangsmaterial wechselt die Bewegungsrichtung in sehr kurzen Intervallen; infolgedessen wird es zerkleinert und mikronisiert.

Die Vorrichtung 10 besteht aus einem aufklappbaren Gehäuse 11 mit einem Materialeinfuhrschacht 11d, in welchem sich zwei Rotorenscheiben 12 befinden, die gegeneinander gestellt sind und mittels entsprechender Motoren 13 über Riemen 13a gegenläufig betrieben werden, so dass sie sich mit gleicher Winkelgeschwindigkeit drehen. Das Gehäuse 11 und die Motoren 13 sind auf dem Fundament 14 befestigt und bilden eine unabhängige Einheit.

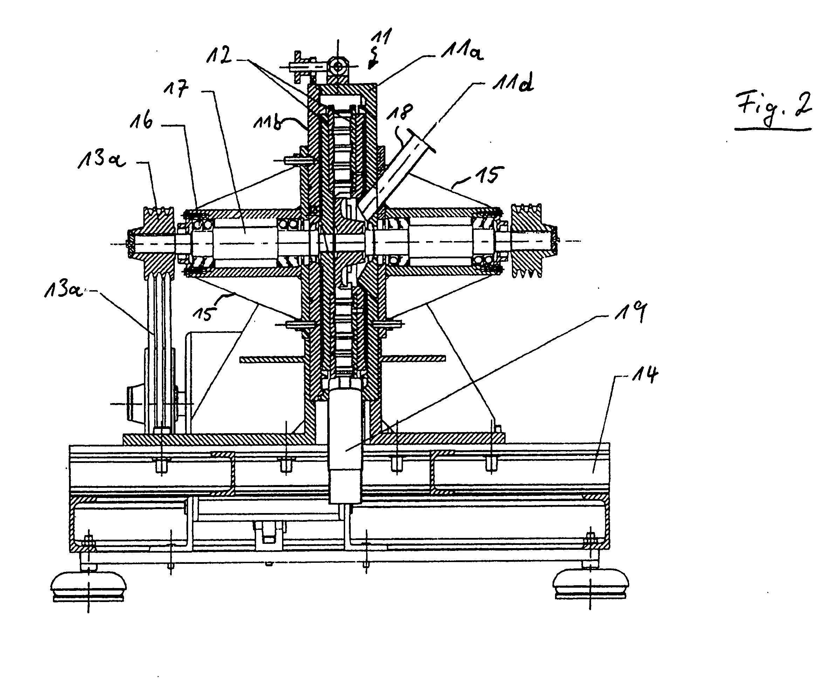

Figur 2 zeigt, dass das Gehäuse 11 aus zwei Teilen aufgebaut ist: eine Gehäuseseite 11 a für die Materialeinfuhr und eine weitere Gehäuseseite 11b mit Einfuhrschacht 11c. Diese zwei Seiten 11a, 11b sind miteinander verschraubt. Auf beiden Seiten des Gehäuses 11 befinden sich die Träger 15, in welche die Lager 16 und Reckstangen 17 eingebaut sind. Auf der Seite 11a für die Materialeinfuhr befindet sich ein Rohr 18 für die geregelte Einfuhr des Materials; auf der unteren Seite befindet sich eine Öffnung 19 für den Ausstoß des fertigen Materials.

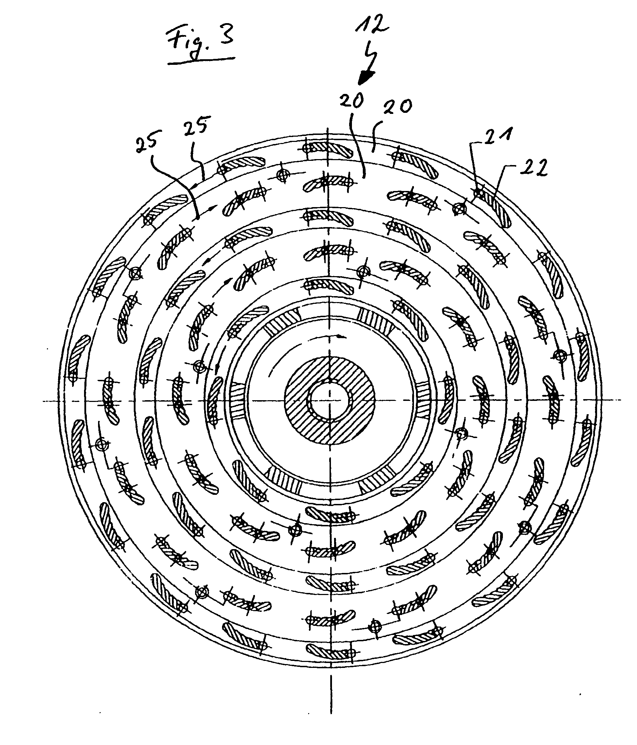

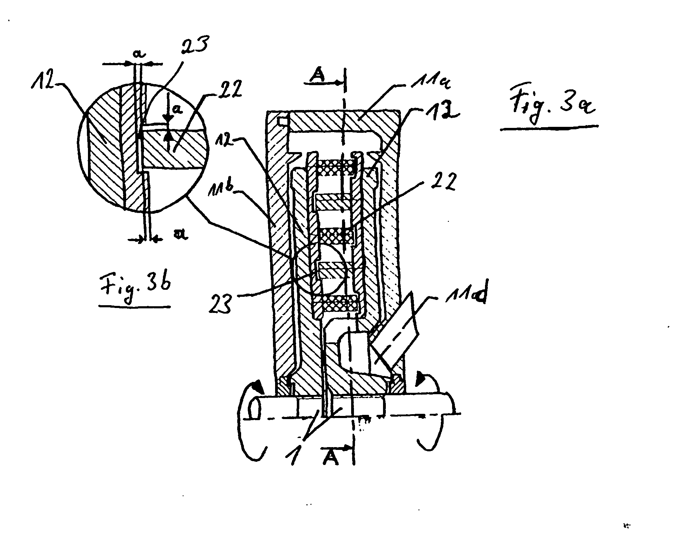

Figur 3, 4 und 5 zeigt, dass auf den Rotorenscheiben 12 mehrere konzentrische Kränze 20 mit den Stiften 21 und Ventilatorschaufeln 22 angeordnet sind, welche so konstruiert und ausgerichtet sind, dass sie berührungsfrei, während sie gegenläufig drehen, nebeneinander vorbeilaufen können - angedeutet durch die Drehrichtung 25. Minimal sind zwei Kränze erforderlich, die von den zwei Rotoren angetrieben werden. Die Aufgabe der Schlagstifte 21. und der Ventilatorschaufeln 22 ist die Erzeugung von turbulenten Luftströmungen für die Beschleunigung des verarbeiteten Materials, so dass Stöße und Reibung unter den Körnchen bei bestimmten Winkeln unter dynamischen Bedingungen hervorrufen werden. Die Kanäle 23 auf den Scheiben verhindern den Materialdurchgang unter den Ventilatorschaufeln 22, vgl. Figur 3 a und 3b.

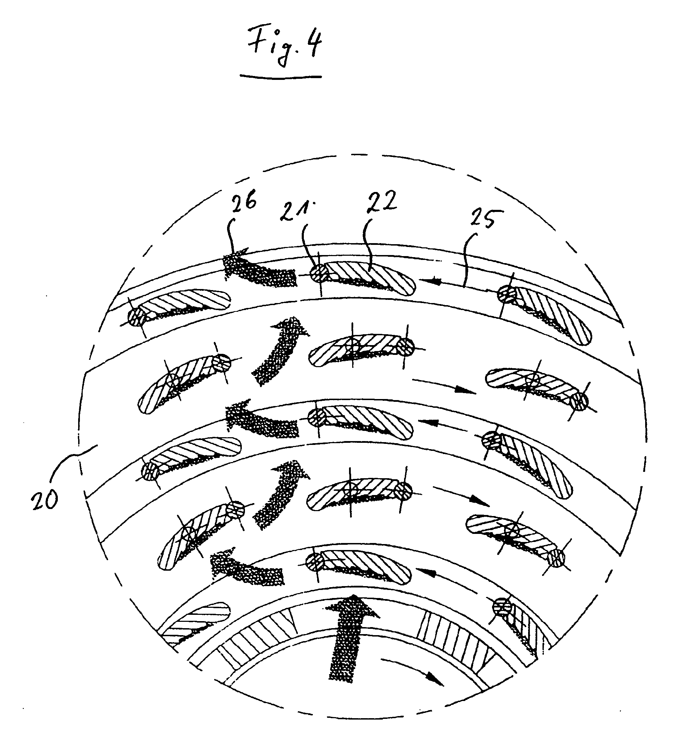

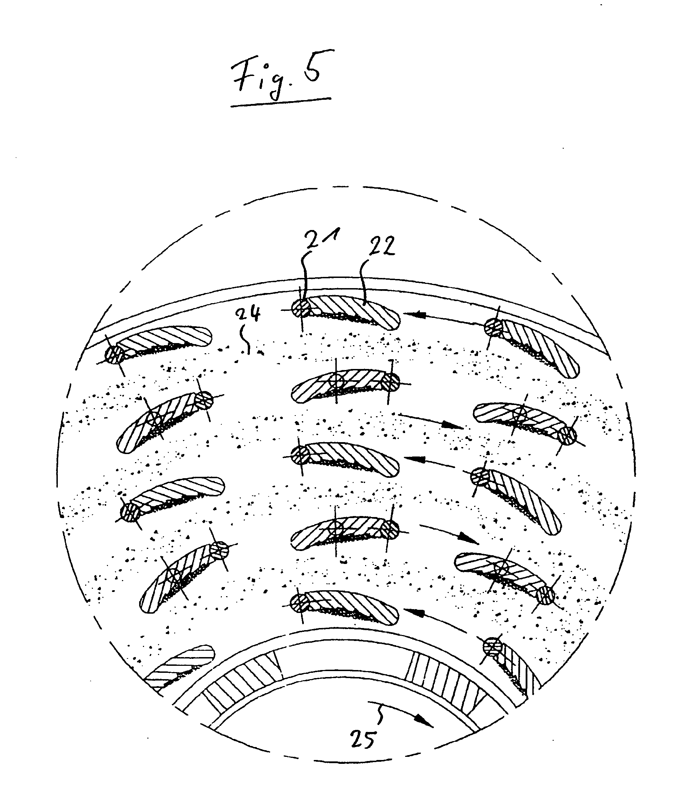

Das Ausgangsgranulat (nicht dargestellt) wird, durch den Zentralteil 18 des Motorensystems durch Einsaugung eingebracht, durch die Luftströme 26 beschleunigt und so gesteuert, dass die Körnchen infolge mehrmaliger Bewegungsrichtung untereinander kollidieren und sich in sehr kurzen Zeitintervallen aneinander reiben. Dabei berühren sich die Arbeitswerkzeuge und andere Teile der Vorrichtung nicht oder nur geringfügig - aber es kommt auf keinen Fall zu Zerstörungen der Werkzeuge. Es entsteht eine Interaktion unter den Körnchen mit solchem Ausmaß, dass bei den Körnchen die innere Energie ausgetauscht wird, da die Zusammenstöße unplastisch sind (vgl. Fig. 4 und 5).

Die Effekte, die Folge der Zusammenstöße der Körnchen sowie Folge der relativen Bewegung der Oberfläche eines Körnchen über der Oberfläche von einem anderen Körnchen (mechanische Reibung) sind, werden verstärkt durch diejenigen Effekte, die infolge plötzlicher Richtungswechsel der Kömchenbewegung entstehen, so dass die Energie der Beschleunigung und relativer Bewegung der Körnchen in Energie der Deformierung, sowie in die Energie der molekularen Bewegung umgewandelt wird. Während der Zusammenstöße und der Reibung der Körnchen, welche dem Verkleinerungsprozeß in sehr kurzen Zeitintervallen (10⁻⁵ bis 10⁻⁶ s) unterzogen sind, entsteht eine bedeutende Veränderung ihrer Geometrie bzw. Form und Größe. Durch die relative Bewegung eines Körnchens über der Oberfläche eines anderen Körnchens, entstehen Schäden und Deformierungen der Körnchenoberfläche, sowie der Materialschichten, welche sich unmittelbar unter der Kornoberfläche befinden. Dadurch wird die Struktur des Kristallgitters auf der Oberfläche zerstört oder geschädigt, so dass teilweise die Kristallform in eine amorphe Phase umgewandelt wird, mit dem Ergebnis, dass die physikalischen und physikalisch-chemischen, sowie energetischen Eigenschaften des verarbeitenden Materials verändert werden. Bei solchen Verarbeitungen von Rohstoffkomponenten organischen Ursprungs zerreißen beispielsweise Zellulosefasern und große Molekülen werden mithin zu kleineren Molekülen, wobei diverse Veränderungen chemischer Zusammensetzung bei dem verarbeiteten Material hervorgerufen werden, sowie auch physikalische Veränderungen, die bedeutend für ihre weitere Materialverarbeitungen und/oder Aufbereitung, aber auch für die Wirksamkeit sind.

Neben der Veränderung der Eigenschaften des Materials, welches mit den beschriebenen Verfahren verarbeitet wird, wird dieses infolge von mechanischen Beanspruchungen fein gemahlen und mikronisiert und die Veränderung der granulometrischen Zusammensetzung des Materials ist von der Granulometrie der Ausgangskörner, sowie vom Niveau der Kömchenbeschleunigung, der geplanten Winkel der Zusammenstöße und gegenseitigen Reibung, sowie die geplanten Anzahl der Zusammenstöße abhängig. Folgende Parameter für die Feinmahlung und Mikronisierung mit der erfindungsgemäßen Vorrichtung stellen eine optimale Konfiguration dar, so dass diese Parameter bevorzugt sind:
■ Granulation des Ausgangskoms < 4,0 mm
■ Anzahl der Gesamtzahl der Kränze/Kranzreihen auf den Rotorenscheiben: 5
■ Durchmesser der Rotoren 500 mm
■ Drehzahl der Rotoren 3600 /Min.
■ Kapazität der Vorrichtung 300Kg/Std.

Verglichen mit den Materialien, welche auf herkömmliche technische Weise (vgl. DE 197 55 921.2) feingemahlen und mikronisiert werden, zeigt das Material, welches durch die erfindungsgemäße Vorrichtung feingemahlen und mikronisiert wurde, eine erhöhte freie Energie und Reaktionsfähigkeit.

Die Innovation liegt mithin in der Konstruktion (Form, Verzahnung, Neigung) und Austauschbarkeit von Stiften und Ventilatorschaufeln auf die konstruierten Kanäle, die sich auf der Rotorenscheibe befinden, sowie in der Auswahl des Materials für die Ausarbeitung von Ventilatorschaufeln. Die Rotationsscheiben der Vorrichtung rotieren mit der gleichen Winkelgeschwindigkeit, bewegen sich jedoch gegenläufig. Das Ausgangsmaterial wird durch das Einsaugrohr 18 in den Zentralteil der rotierenden Scheibe eingeführt, aufgrund von Zentrifugalkräften werden die Körnchen der Rohstoffkomponente in Richtung des äußeren Randes des Gehäuses beschleunigt. Die Körnchen schlagen auf die Kränze 20 der Ventilatorschaufeln 22, welche sich in Gegenrichtung drehen. Sie wechseln die Bewegungsrichtung aufgrund der Richtungswechsel der Ventilatorschaufeln. Ferner schlagen und reiben sich die Körnchen untereinander, gehen nachfolgend in einen weiteren Kranz 20 mit Ventilatorschaufeln 22 über, wechseln wieder die Richtung der Bewegung im Einklang mit dem Richtungswechsel der rotierenden Scheibe, solange bis sie das Schaufelsystem verlassen. Am Ende der Bearbeitung in der erfindungsgemäßen Vorrichtung schlagen die Körnchen auf der Wand des Gehäuses auf und werden durch die Ausgangsöffnung 19 abtransportiert.

Figur 3a und 3b zeigt, dass die Kanäle 23 auf den Scheiben 12, in welche die Ventilatorschaufeln 22 durchgreifen, den Materialdurchgang unter den Ventilatorschaufeln 22 verhindern. Deren Form wird gemäß den Eigenschaften der jeweils zu verarbeitenden Rohstoffkomponenten (Materials) - also Granulation des Ausgangsmaterials, seine Feuchtigkeit, Härte, Ursprung, chemische Zusammensetzung und ähnliches - definiert. Wenn z. B. die Eingangsgranulation des Materials < 1 mm beträgt, bedeutet dies, dass der minimale Abstand zwischen den Ventilatorschaufeln und den Kanälen auf den Scheiben größer als 1 mm sein soll, um überhaupt das Durchgehen des Materials zu ermöglichen. Bei der Ausarbeitung und der Montage der Scheiben soll zufriedenstellende Parallelität verwirklicht werden, um den Ventilatorschaufeln das Durchgreifen in die Kanäle zu ermöglichen, denn die Durchmesser der Scheiben sind relativ groß (500 mm). Vorteile gegenüber der in der DE 197 55 921.1 beschriebenen Vorrichtung liegen darin, dass die Ventilatorschaufeln aufgrund ihrer Form, Neigung und Verzahnung bei dem Prozeß der Feinmahlung und Mikronisierung mit einer feinen Schicht des verarbeiteten Materials belegt werden und auf diese Weise von der Schlag- und Reibungswirkung des Eingangsmaterials geschützt werden. Die Abnutzung der Oberfläche der Ventilatorschaufeln wird somit minimiert und die Lebensdauer bedeutend verlängert. Ferner wird die energetische Ladung des Materials, welches behandelt wird, angehoben, wenn die Körnchen untereinander kollidieren und nicht mit den Teilen der Ventilatorschaufeln. Die technologischen Parameter, wie die Anzahl der Ventilatorschaufeln, ihre Neigung, die Form der Schaufelverzahnung, Anzahl der Ventilatorkränze, Winkelgeschwindigkeit der Scheiben, definieren die späteren Eigenschaften des verarbeiteten Materials. Mit der Kombination der angeführten Parameter wird es möglich, die Ergebnisse und Effekte zu programmieren.

In der Figur 3b sind weiter Scheibenkanäle (23) näher dargestellt. In den Rotorenscheiben (12) befinden sich an denjenigen Orten, die mit der Kranzreihe (20) des gegenüberliegenden Rotors (13) korrespondieren, Ausnehmungen (20a; siehe Fig. 3a, 3b), innerhalb derer die Ventilatorschaufeln (22) des gegenüberliegenden Kranzes (20) laufen. Die Kanäle (23) decken sich gegenseitig mit der Länge a, welche 2 - 5 mm beträgt. Sie bilden quasi ein Labyrinth, welches den Durchgang des Materials unter den Ventilatorschaufeln verhindert. Durch die Entstehung des Labyrinthes verstärkt sich der Widerstand für die Strömung unter den Ventilatorschaufeln. Somit wird erreicht, dass sich die Körnchen des Ausgangsmaterials durch die Hauptströmung zwischen den Ventilatorschaufeln bewegen. Die Form der Kanäle wird gemäß technisch-technologischer Eigenschaften der Rohstoffkomponenten des verarbeiteten Materials definiert (Granulation des Ausgangsmaterials, seine Feuchtigkeit, Härte, Ursprung, chemische Zusammensetzung und ähnliches). Wenn es die Scheibenkanäle nicht geben würde, würde sich das Ausgangsmaterial aufgrund der Zentrifugalkräfte so bewegen, dass es vom Zentrum bis zu der Peripherie der Scheiben an den Ventilatorschaufeln und der Scheibe vorbeigeht. Bei der Ausgangsgranulation des Materials von 0 - 1 mm muß die Entfernung zwischen den Ventilatorschaufeln und den Kanälen größer als 1 mm sein, um den Durchgang des Materials überhaupt zu ermöglichen, vorteilhaft hat sich eine Entfernung von 2 mm genauso wie für die Verdeckung a erwiesen.

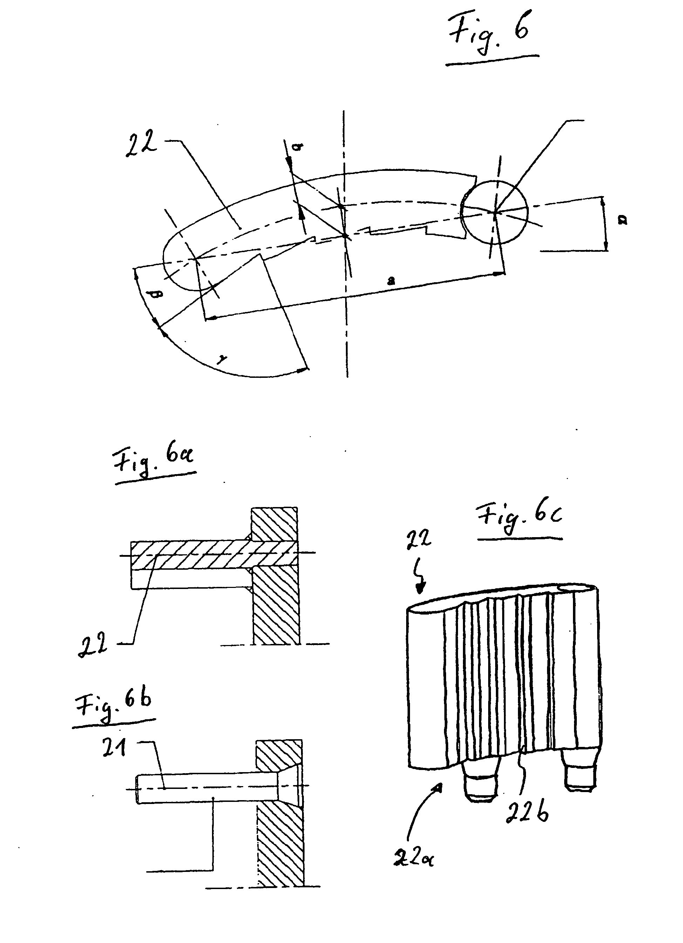

Die Figuren 6, 6a, 6b und 6c zeigen die Geometrie der Ventilatorschaufeln 22 und der Stifte 21. Der Kranz 20, die Ventilatorschaufeln 22 und die Stifte 21 sind aus Hartstahl gebaut. Eine Alternative ist, den Stift aus Porzellan und die Ventilatorschaufeln aus Stahl herzustellen. Die Neigung der Ventilatorschaufeln 22 beträgt im Verhältnis zur Horizontalen α = 4 - 15° mit der Optimierung auf 8 - 10°. Die Anordnung und die Größe von der Verzahnung ist von der Anzahl der Ventilatorschaufeln abhängig (β = 30 - 120° und γ = 60 - 120°). Der Biegungsgrad der Ventilatorschaufeln ist mit dem Verhältnis der Längen a und b definiert, wobei b 10 % der Sehnenlänge darstellt. Die Anordnung der Ventilatorschaufeln ist fixiert. Sie sind in die Kranzform in entsprechende Ausnehmungen eingepreßt Die Form der Ventilatorschaufeln, profilierte Kollisionsoberfläche 22a, 22b und die Neigung sichern die Abfüllung der Ventilatorschaufeln mit dem Ausgangsmaterial und werden damit von der Abnutzungswirkung des Ausgangsmaterials geschützt, was auf die Verlängerung der Lebensdauer der Ventilatorschaufeln wirkt. Die Zähne 22b auf den Ventilatorschaufeln halten die erste Schicht des Ausgangsmaterials auf der Kollisionsoberfläche 22a, während die zweite Schicht langsam über die erste Schicht rutscht und diese zweite Schicht nimmt die Schläge des ankommenden Ausgangsmaterials an. Bevor die Ventilatorschaufeln 22 in die jeweilige Kranzreihe 20 der Rotorenscheibe 12 eingebracht wird, wird der Stift 21 in die Rotorenscheibe 12 eingepreßt. Die Symmetrieachse des Stiftes 21 befindet sich in der Symmetrieachse des Biegungsgrades der Ventilatorschaufeln 22, damit die Geometrie des Systems der Ventilatorschaufeln/Stifte optimal erreicht wird. Während der Materialverarbeitung schlagen die Materialkörnchen auf die Stirnseite der Ventilatorschaufeln und insbesondere auf und in die Stifte. Der Stift wird somit abgenutzt. Nach der Abnutzung wird der Stift durch Auspressen oder Bohren durch einen neuen ausgetauscht. Dies hat einen entscheidenden Vorteil: Bisherige Varianten der Ventilatorschaufeln ohne Stifte haben die Abnutzung der Ventilatorschaufeln selbst ergeben. Der Austausch der abgenutzten Ventilatorschaufeln ist im Vergleich mit dem Austausch der Stifte sehr kompliziert, aufwendig und teuer. Die Stifte können daher leicht ausgetauscht werden; die Ventilatorschaufeln bleiben unbeschädigt und müssen es nicht. De Austausch der Ventilatorschaufein erfolgt erst bei allgemeiner Materialermüdung.

Das Problem der Vibrationen und der Festigkeit der Stifte ist dadurch gelöst, dass sich der Stift 21 mit der Fläche von 1/3 seines. Stiftumfanges an eine korrespondierende Ausnehmung der Ventilatorschaufel 22 an diese Ventilatorschaufeln "anlehnt", und sich nicht geradflächig an die Ventilatorschaufeln anlehnt. Die Ventilatorschaufeln 22 werden durch Kaltpressen verformt oder auch durch Schmieden, wobei das Schmieden der besseren Aushärtung dient.

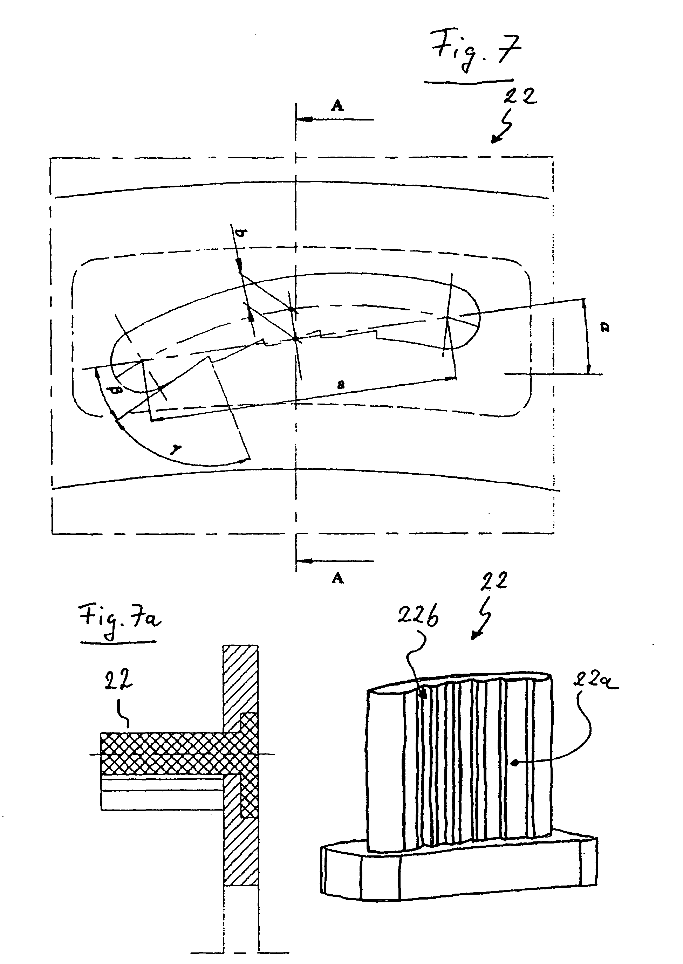

Eine andere Ausführungsform sieht erfindungsgemäß auch Segment-Ventilatorschaufeln (Figur 7, 7a) vor. Die Segment-Ventilatorschaufeln 22 werden aus Keramik oder Gußstahl hergestellt. Bei dieser Ausführungsform werden in die Rotorenscheiben 12 die Kanäle eingebaut, in welche die Segment-Ventilatorschaufeln mit unbestimmten Einlegungen gestellt werden. Die Genauigkeit der Einstellungen und die Festigkeit der Ventilatorschaufeln wird durch das Profil der Kanäle und durch die Reibung zwischen den Segment-Ventilatorschaufeln und die tragende Scheibe bestimmt. Die Neigung der Ventilatorschaufeln in Bezug auf die Horizontale beträgt α = 4 - 15° mit der Optimierung bei 8° - 10°. Die Einordnung der Zähne 22b und ihre Größe ist von der Länge der Ventilatorschaufeln 22 abhängig, Winkel β beträgt 30° - 120°, und Winkel γ beträgt 60° - 120°. Der Biegungsgrad der Ventilatorschaufeln ist mit dem Verhältnis der Längen a und b definiert, wobei b 10 % der Sehnenlänge darstellt. Die Form der Ventilatorschaufeln, der profilierten (verzahnten) Kollisionsoberfläche und der Neigung sichern, dass die Ventilatorschaufeln mit dem Ausgangsmaterial abgefüllt werden und damit von der Abnutzungswirkung des Ausgangsmaterials geschützt werden, was auf die Verlängerung der Lebensdauer der Ventilatorschaufeln wirkt. Die Zähne 22b auf den Ventilatorschaufeln halten wie in Figur 6 die erste Schicht des Ausgangsmaterials auf der Kollisionsoberfläche 22a, während die zweite Schicht langsam über die erste Schicht rutscht und diese zweite Schicht nimmt die Schläge des ankommenden Ausgangsmaterials an. Diese Ausführung hat im Vergleich zu obiger Ausführung (Fig. 6) den Vorteil, dass die Ventilatorschaufeln mit verschiedenen Neigungen (Winkel α) eingebaut werden können; somit ist der Austausch der Ventilatorschaufeln einfacher. Die Form der Ventilatorschaufeln hat keine Aussparung für die Stifte.

### Bezugszeichen

- 10: Vorrichtung
- 11: Gehäuse
- 11a: Gehäuseseite zur Materialeinfuhr
- 11b: Gehäuseseite mit Verschlußdeckel
- 11c: Verschlußdeckel
- 11d: Materialeinfuhrschacht
- 12: Rotorenscheiben
- 13: Motoren
- 13a: Riemen
- 14: Fundament / Gestell
- 15: Träger
- 16: Lager
- 17: Reckstangen
- 18: Einfuhrrohr
- 19: Auslaß
- 20: Kränze
- 20a: Ausnehmung
- 21: Stifte
- 22: Ventilatorschaufeln
- 22a: Kollisionsfläche
- 22b: Verzahnung
- 23: Kanäle
- 24: Zermahlenes Material
- 25: Drehrichtung
- 26: Luftströme

## Patentansprüche

1. Verwendung von mikronisierten Zeolithen als Filterzusatzstoffe bei Filtrationsprozessen, wobei der Korngrößendurchmesser der mikronisierten Zeolithe unter 0,5 µm liegt, **dadurch gekennzeichnet, dass** die verwendeten Zeolithe durch Rotoren, die aus Scheiben bestehen, auf denen die Ventilatorschaufeln einseitig befestigt angeordnet sind und wobei die Ventilatorschaufeln mit den Kränzen verbunden sind und in korrespondierende Kanäle auf der jeweils gegenüberliegenden Rotorenscheibe durchgreifen, die einen Materialdurchgang unter den Ventilatorschaufeln verhindern, zerkleinert wurden.

2. Verwendung von mikronisierten Zeolithen als Filter-Zusatzstoffe bei Filterzigaretten, **dadurch gekennzeichnet dass** der Korngrößendurchmesser der mikronisierten Zeolithe unter 0,5 µm, insbesondere zwischen 0,2 µm und 0,5 µm, liegt, welche durch Rotoren, die aus Scheiben bestehen, auf denen die Ventilatorschaufeln einseitig befestigt angeordnet sind und wobei die Ventilatorschaufeln mit den Kränzen verbunden sind und in korrespondierende Kanäle auf der jeweils gegenüberliegenden Rotorenscheibe durchgreifen, die einen Materialdurchgang unter den Ventilatorschaufeln verhindern, zerkleinert wurden.

3. Verwendung nach Anspruch 2, **dadurch gekennzeichnet, dass** die mikronisierten Zeolithe direkt auf die Zellulose-Acetat-Faser des Filters oder das Papier bzw. Crest aufbracht werden.

4. Verwendung nach Anspruch 3, **dadurch gekennzeichnet, dass** bis zu 11,3 mg mikronisierte Zeolithe pro Filter, bevorzugt 0,5 mg pro Filter, verwendet werden.

5. Verwendung nach wenigstens einem der Ansprüche 2 bis 4, **dadurch gekennzeichnet, dass** Selen in einer Konzentration von bis zu 25 µg pro Filter zugegeben wird.

## Claims

1. Use of micronized zeolites as filter additives in filtration processes, the grain diameter of said micronized zeolites being below 0.5 µm, **characterized in that** the zeolites used have been reduced in size by means of impellers constituted of disks having the ventilator blades arranged thereon, mounted on one side, said ventilator blades being connected with the rims, extending into corresponding channels on each opposing impeller disk, which prevent passage of material beneath the ventilator blades.

2. Use of micronized zeolites as filter additives in filter cigarettes, **characterized in that** the grain diameter of the micronized zeolites is below 0.5 µm, particularly between 0.2 µm and 0.5 µm, which zeolites have been reduced in size by means of impellers constituted of disks having the ventilator blades arranged thereon, mounted on one side, said ventilator blades being connected with the rims, extending into corresponding channels on each opposing impeller disk, which prevent passage of material beneath the ventilator blades.

3. The use according to claim 2, **characterized in that** the micronized zeolites are directly applied to the cellulose acetate fiber of the filter or to the paper or Crest.

4. The use according to claim 3, **characterized in that** up to 11.3 mg of micronized zeolites per filter, preferably 0.5 mg per filter, are employed.

5. The use according to at least one of claims 2 to 4, **characterized in that** selenium is added at a concentration of up to 25 µg per filter.

## Revendications

1. Utilisation de zéolithes micronisées comme additif de filtration dans des procédés de filtration, le diamètre des particules de zéolithe micronisée étant inférieur à 0,5 µm, **caractérisée en ce que** les zéolithes utilisées ont été broyées à l'aide de rotors constitués de disques sur une des faces desquels sont fixées des ailettes de ventilateur, et les ailettes de ventilateur sont reliées aux couronnes et traversent les canaux correspondants du disque de rotor opposé, ce qui empêche le passage de matières sous les ailettes de ventilateur.

2. Utilisation de zéolithes micronisées comme additif de filtration dans des filtres de cigarettes, **caractérisée en ce que** le diamètre des particules de zéolithe micronisée est inférieur à 0,5 µm, de préférence, comprise entre 0,2 µm et 0,5 µm, que ces zéolithes ont été broyées à l'aide de rotors constitués de disques sur une des faces desquels sont fixées des ailettes de ventilateur, et les ailettes de ventilateur sont reliées aux couronnes et traversent les canaux correspondants du disque de rotor opposé, ce qui empêche le passage de matières sous les ailettes de ventilateur.

3. Utilisation selon la revendication 2, **caractérisée en ce que** les zéolithes micronisées sont déposées directement sur les fibres en acétate de cellulose du filtre ou sur le papier resp. embout.

4. Utilisation selon la revendication 3, **caractérisée en ce que** l'on utilise jusqu'à 11,3 mg de zéolithe micronisée par filtre, de préférence 0,5 mg par filtre.

5. Utilisation selon au moins une des revendications 2 à 4, **caractérisée en ce que** du sélénium est ajouté en une concentration allant jusqu'à 25 µg par filtre.
